# EUROPEAN PATENT SPECIFICATION

(11) **EP 1 846 044 B1**
(45) Date of publication and mention of the grant of the patent: **11.03.2009**
(21) Application number: 06700019.0
(22) Date of filing: 06.01.2006
(51) Int. Cl.: A61L 9/12

(54) **MULTIPLE VOLATILE LIQUIDS DISSEMINATING DEVICE**
VORRICHTUNG ZUR VERTEILUNG MEHRERER FLÜCHTIGER FLÜSSIGKEITEN
DISPOSITIF DE DISSÉMINATION DE PLUSIEURS LIQUIDES VOLATILS

(30) Priority: 12.01.2005 GB 0500481
(43) Date of publication of application: 24.10.2007
(73) Proprietor: Givaudan SA, 1214 Vernier-Genève (CH)
(72) Inventor: BROWN, Colin, Bracknell Berkshire RG42 2BD (GB); NAISH, Guy Edward, Bicester Oxfordshire OX26 3WE (GB); GOHIL, Kishen, New Malden Surrey KT3 6HB (GB)
(74) Representative: Givaudan Patents
(86) International application number: PCT/CH2006/000011
(87) International publication number: WO 2006/074562

(56) References cited:
- EP-A- 0 546 214
- WO-A-97/02076
- WO-A-20/04096300
- DE-A1- 4 135 796
- FR-A- 2 841 081
- US-A- 1 749 187
- US-A- 2 562 959
- US-B1- 6 357 726

## Description

This invention relates to apparatus for disseminating volatile liquids in vapour form into an atmosphere.

Apparatus for dissemination of a volatile substance, such as a fragrance, an odour masking agent or an insecticide, into an atmosphere are well known and widely used. These apparatus are generally simple and therefore cheap to manufacture, but they are universally for single fragrances. They are inadequate for more sophisticated applications where a number of fragrances are required, especially when it is desirable that fragrances be selectable.

There already exist on the market apparatus that can disseminate a number of fragrances and that permit the selection of which fragrance will be disseminated at which time. They generally involve a fan and a number of containers, the containers having suitable disseminating members (such as porous wicks allowing the transport of the liquid between a storage reservoir and the atmosphere) and being movable with respect to the fan. Thus, when a particular fragrance is desired, the appropriate container and disseminating member is moved into position with respect to the fan, such that the air current from the fan evaporates the volatile liquid and conveys it to the atmosphere.

While such apparatus are generally satisfactory in performance, they have several drawbacks. One is that the mechanism for moving the various containers is complex and therefore expensive and more prone to mechanical failure. Another drawback is that such an apparatus requires the use of fragrances in a particular sequence, the one in which they are mounted. It is possible to utilise fragrances out of sequence, but this involves a further undesirable degree of mechanical and electrical complexity.

EP 0 546 214 discloses an air freshener with radially configured scent reservoirs, which are selectively passed by an air current.

WO 2004/096300 discloses an air freshener comprising a fan coplanar with two scent reservoirs; the flow path is switchable by a movable flow controller and the fan, which may change its rotation sense.

Further attempts to overcome these and similar problems have resulted in sophisticated apparatus. Examples of such apparatus include Japanese Kokai H03-139630 and Kokai 2003-310740, UK published application GB 2 041 407 and PCT published application WO 2003/028775. All of these are relatively complex and therefore relatively expensive.

It has now been found that a relatively simple, robust apparatus can be used to disseminate a plurality of individual volatile liquids into an atmosphere. The invention therefore provides an apparatus according to claim 1.

The invention further comprises a method according to claim 8.

The source of air current may be any suitable source, typically a fan, vane or an impeller (hereinafter generally referred to as "fan"), rotated by any suitable means, for example, by an electric motor on whose shaft it is mounted. The motor may be powered by any suitable power source, such as battery, mains electricity or solar cells. In one aspect of the invention, the fan is one that causes an air current that is coplanar with the fan, that is, radial from the axis of rotation of the fan.

The plurality of channels is positioned so as to be in the air current. In the embodiment referred to hereinabove in which the air current is coplanar with the fan, the channels are placed radially around the circumference of the fan, preferably equidistant from each other. The number of channels will depend on the number of volatile liquids desired, but it is at least three, as known simpler devices can easily manage two volatile liquids. A typical number is between 3 and 8, and in a further aspect between 4 and 6; this permits a desirable number of volatile liquids along with a desirable combination of reasonable liquid quantity of internal reservoirs and compact dimensions. It is possible of course to have larger reservoirs remote from the apparatus and supplying the apparatus by means of feeder tubes. This increases the number of possible volatile liquids substantially, but it also increases considerably the complexity of the apparatus, and while such an apparatus may be appropriate for large institutions, it is not appropriate for most uses, where a relatively simple, self-contained, easily replenished apparatus is desirable.

Thus, in one particular embodiment, the volatile liquids are held in replaceable reservoirs, allowing for easy replenishment.

In the embodiment in which the air current is coplanar with the fan, the channels form part of a body that surrounds at least a substantial part (or even all) of the circumference of the fan. They may be provided by any suitable means in any suitable material, for example, by casting, machining and moulding a material such as a plastic, a metal or a ceramic.

Each channel leads to a volatile liquid-emitting member and from there into the atmosphere. The volatile liquid-emitting member may be any suitable such member. For example, it may be a spray nozzle. However, for simplicity and cheapness, there is used an evaporative surface, such as a porous wick of any suitable material, with one end in a reservoir of volatile liquid and the other in the air stream. Such wicks are well known to the art and are widely available. Alternatively, the liquid-emitting member may be a flat pad of any suitable material, connected to a reservoir of volatile liquid. The volatile liquid may be transferred to the volatile liquid -emitting member by any convenient means, one such means being by capillary action.

The reservoirs and/or the volatile liquid-emitting members may optionally be equipped with heating elements, which assist in the evaporation of liquid into the air stream.

In each channel, between the source of air current and the volatile liquid-emitting member, is provided a means of blocking the channel. This can take any suitable form, and the skilled person will recognise the many kinds of suitable blocking means that are possible. Without limiting the scope of the invention in any way, one advantageous blocking means is electromagnetic in operation. Typical examples include a solenoid valve and an electromagnet that, when switched on, attracts a ferromagnetic material, causing it to move and thus to block (or unblock) a channel. Reopening of the channel may also be by any convenient means, such as gravity acting on a ferromagnetic element that has been raised against the force of gravity by an electromagnet, the urging of a compressed spring or the actuation of a solenoid valve.

The actuation and control of the blocking means and other aspects of the apparatus, for example, the air current speed, may be by any suitable means, and again the skilled person will see the many possibilities within the skill of the art. For example, it may be achieved by means of a pre-set program in the apparatus itself. This may be completely automatic, or it may be capable of being overridden manually by adjusting the apparatus directly, for example, by means of a suitable control panel provided in the apparatus. Such a system may allow a variety of possibilities, such as fragrance selection, order and time of emission and so on. All of these are well within the skill of the art.

Alternatively, actuation and control may be achieved by remote control. One such means of control is a control cable leading from a control unit to the apparatus. The control unit may be a computer or may have a computer associated with it, and instructions may be input into the computer, either directly or via the Internet. However, the control may be wireless, for example, by electromagnetic signal, such as an infra-red apparatus of the type used for television and other domestic entertainment electronic equipment. However, it may also be radio frequency equipment. Such a control unit may be equipped with any necessary control means, for example, means for opening and closing any blocking means or combination of blocking means, means for adjusting the speed of rotation of the air current and means for adjusting the temperature of any heating element. These can all be achieved by readily-available components. The instructions may come, for example, from a dedicated controller, or they may come to a computer equipped with a radio transmitter, either by direct input or over the Internet, or by means of an SMS message from a mobile telephone.

The apparatus according to this invention may be easily manufactured using common materials and techniques. It is versatile and reliable in use, easy to replenish, and it allows the emission into an atmosphere of a selected volatile liquid or combination of such liquids. Any volatile liquid useful for dissemination into an atmosphere in vapour form may be used. The most significant group of such liquids are fragrances, and the apparatus according to this invention can be used to change the fragrance, and therefore the atmosphere or "mood" of a room. Another possible use is use with an audio-visual display, in which a fragrance can be released to match what is on the screen. However, other liquids can also be used, for example, disinfectants, insecticides and fungicides. For example, an apparatus can contain both an insecticide and a fragrance, such that the insecticide release can be remotely triggered, and when that has had sufficient time to work, a fragrance can be released to cover the odour of the insecticide.

The invention is further described with reference to any one of the drawings which depict preferred embodiments and which are not intended to be limiting in any way.
Figure 1 depicts a transverse cross-section through an apparatus.
Figure 2 depicts a perspective exploded view of the embodiment of Figure 1.
Figure 3 is a schematic representation of one form of blocking means.
Figure 4 is a schematic representation of another form of blocking means.

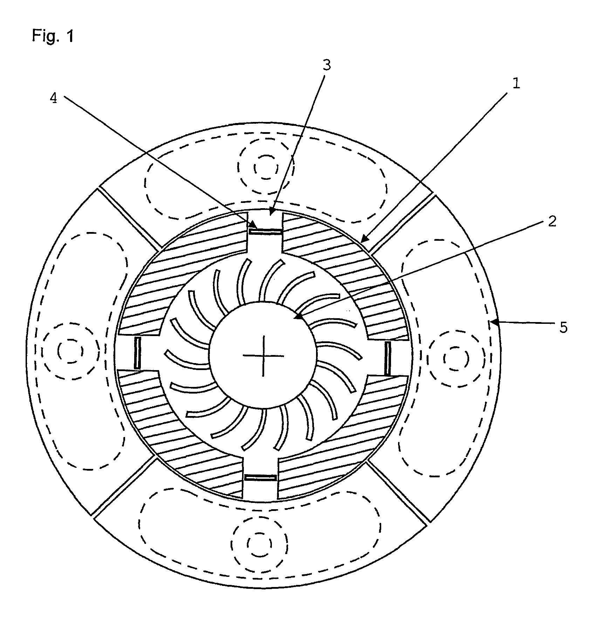

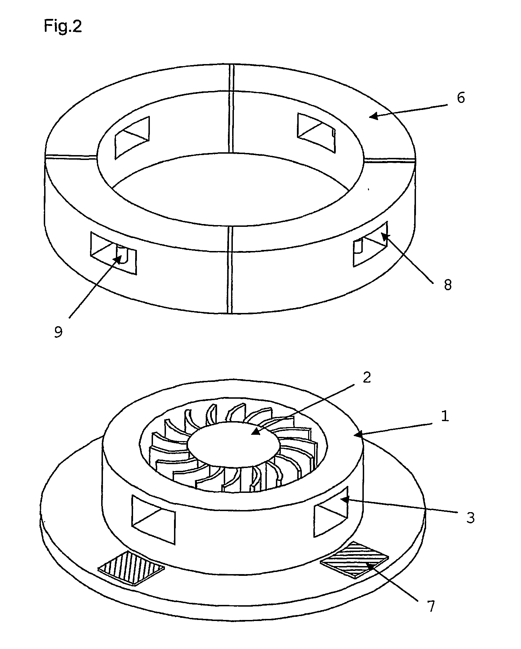

In Figures 1 and 2, an essentially toroidal member 1 surrounds a centrifugal fan 2 and lies in the plane of rotation of the fan. In the toroidal member are formed four channels 3, arranged equidistantly around the toroidal member and extending radially from the fan. Within each channel 3 is a blocking means 4. Surrounding the toroidal member is a further toroidal member 6, which has four channels 8 that form continuations of the four channels 3. Within the further toroidal member 6 are four reservoirs of fragrance (shown in ghosted outline at 5) with associated fragrance-emitting means 9, in this case porous wicks leading from the reservoir and protruding into the channel 8. The toroidal member 1 also has mounted thereon heating elements 7, one for each reservoir, such that, when the apparatus is assembled, each reservoir 5 sits on a heating element. If desired, the heating element can be switched on and assist in the transport and evaporation of the fragrance.

In operation, the fan 2 blows air radially outwards. The air is blown through the channels 3, and, if the blocking means 4 for the particular channel is open, past the wick 9, where it evaporates and entrains fragrance, and then through channel 8 into the atmosphere.

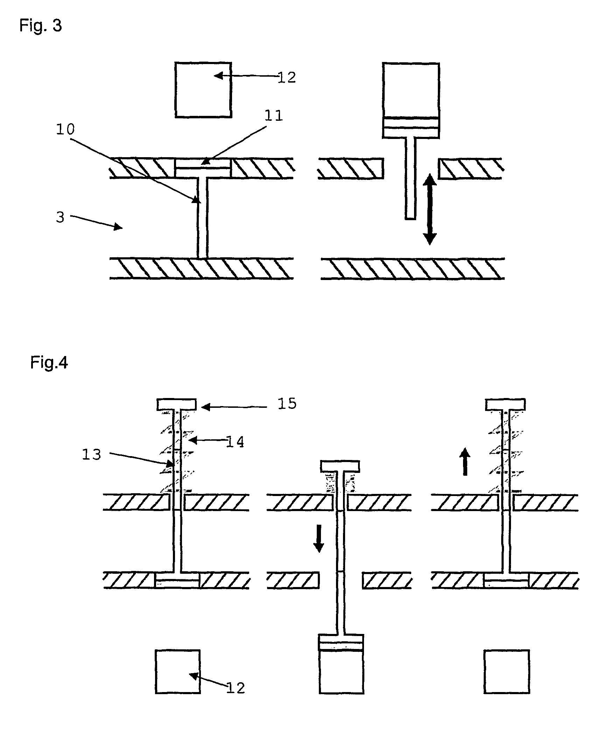

In Figure 3, the blocking means has the form of a gate valve 10, which blocks the channel 3 and which is able to be lifted vertically to such an extent that it clears the channel 3. At the top of the valve is a piece of ferromagnetic material (typically steel) 11, which is in close proximity to an electromagnet 12. When the electromagnet 12 is energised, the ferromagnetic material is attracted to it, and the gate valve is thus raised and the channel 3 opened. When the electromagnet is no longer energised, gravity causes the gate valve to close.

Figure 4 depicts a variant of the Figure 3 valve. In this case, the gate valve is attracted downwards by the electromagnet 12 and it has a port 13 that is brought into alignment with the channel by the downwards movement. It is closed again by means of a helical spring 14 fitted to an elongated stem of the gate valve and which seeks to pull the gate valve upwards and away from the electromagnet. It does this when the electromagnet is no longer energised.

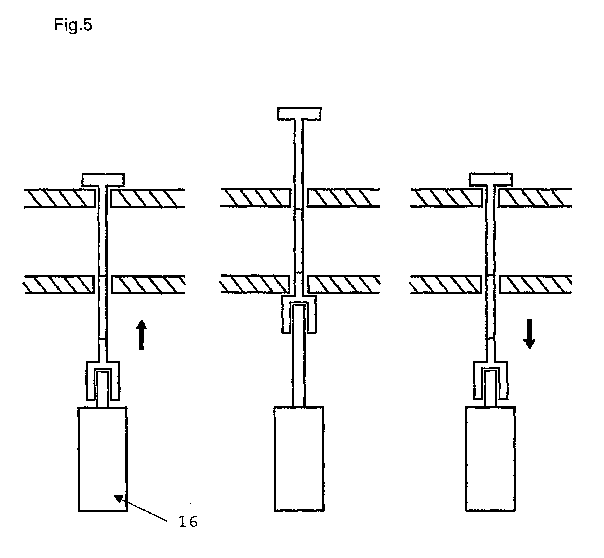

The valve of Figure 5 is a variation on that of Figure 4, in this case being operated by a solenoid switch 16 positioned beneath the gate valve. When the switch is activated, it pushes the gate valve upwards, this bringing the valve port into alignment with the channel, thus opening it. When it is deactivated, the switch returns to its rest position and the gate closes.

## Claims

1. An apparatus adapted to provide in an atmosphere one of at least three volatile liquids, the apparatus comprising a source of air current (2) adapted to convey the individual liquids into the atmosphere,
wherein
(a) the source of air current is a fan or impeller;
(b) there extend radially from the fan or impeller a plurality of air channels ((3) & (8)), **characterised in that** said air channels (3) are coplanar with the plane of rotation of the fan or impeller, corresponding to the number of volatile liquids, placed such that the air current flows through them, each channel leading to a different volatile liquid-emitting member (9) and then into the atmosphere; and
(c) there is located in each channel between the source and the volatile liquid-emitting member, means (4) for blocking and reopening the channel and preventing the flow of air therethrough.

2. An apparatus according to claim 1, wherein the number of channels ((3) & (8)) is from 3-8, preferably from 4-6.

3. An apparatus according to claim 1, wherein the liquid-emitting member (9) comprises an evaporative surface, preferably a wick protruding into the path of the air current.

4. An apparatus according to claim 1, wherein the blocking means (4) are electromagnetically actuated.

5. An apparatus according to claim 4, wherein the blocking means are doors operated by solenoid switches (16).

6. An apparatus according to claim 1, wherein all functions of the apparatus are regulable directly by control means on the apparatus.

7. An apparatus according to claim 1, wherein all functions are regulable remotely, preferably by radio transmission.

8. A method of disseminating into an atmosphere at least one volatile liquid selected from at least three such liquids provided in the apparatus according to claims 1-7, comprising the passing of an air current from the source of air current (2) through the plurality of air channels ((3) & (8)) corresponding in number to the number of volatile liquids.

## Patentansprüche

1. Vorrichtung, die geeignet ist, eine von mindestens drei flüchtigen Flüssigkeiten an die Atmosphäre abzugeben, und eine Luftstromquelle (2) umfasst, die geeignet ist, die einzelnen Flüssigkeiten in die Atmosphäre zu befördern,
wobei
(a) es sich bei der Luftstromquelle um ein Gebläse oder Gebläserad handelt,
(b) sich mehrere Luftkanäle ((3) & (8)) radial vom Gebläse oder Gebläserad erstrecken, **dadurch gekennzeichnet, dass** die Luftkanäle (3) zu der Rotationsebene des Gebläses oder Gebläserads koplanar sind, der Anzahl flüchtiger Flüssigkeiten entsprechen und so platziert sind, dass der Luftstrom durch sie hindurchströmt, wobei jeder Kanal zu einem anderen flüchtige Flüssigkeit emittierenden Element (9) und dann in die Atmosphäre führt, und
(c) in jedem Kanal zwischen der Quelle und dem flüchtige Flüssigkeit emittierenden Element Mittel (4) angeordnet sind, um den Kanal zu blockieren und wieder zu öffnen und den Luftstrom dort hindurch zu verhindern.

2. Vorrichtung nach Anspruch 1, wobei die Anzahl von Kanälen ((3) & (8)) 3 - 8, vorzugsweise 4 - 6, beträgt.

3. Vorrichtung nach Anspruch 1, wobei das flüssigkeitsemittierende Element (9) eine Verdunstungsfläche, vorzugsweise einen in den Weg des Luftstroms vorragenden Docht, umfasst.

4. Vorrichtung nach Anspruch 1, wobei die Blockiermittel (4) elektromagnetisch betätigt werden.

5. Vorrichtung nach Anspruch 4, wobei es sich bei den Blockiermitteln um Türen handelt, die durch Magnetschalter (16) betätigt werden.

6. Vorrichtung nach Anspruch 1, wobei alle Funktionen der Vorrichtung durch Steuermittel an der Vorrichtung direkt reguliert werden können.

7. Vorrichtung nach Anspruch 1, wobei alle Funktionen abgesetzt, vorzugsweise per Funkübertragung, reguliert werden können.

8. Verfahren zur Verteilung von mindestens einer flüchtigen Flüssigkeit in der Atmosphäre, die aus mindestens drei derartigen in der Vorrichtung nach Ansprüchen 1 - 7 vorgesehenen Flüssigkeiten ausgewählt ist, wobei ein Luftstrom von der Luftstromquelle (2) durch die mehreren Luftkanäle ((3) & (8)), deren Anzahl der Anzahl flüchtiger Flüssigkeiten entspricht, geführt wird.

## Revendications

1. Appareil prévu pour fournir, dans une atmosphère, un parmi au moins trois liquides volatils, l'appareil comprenant une source de courant d'air (2) prévue pour transporter les liquides individuels dans l'atmosphère,
(a) la source de courant d'air étant un ventilateur ou une hélice ;
(b) une pluralité de canaux d'air (3 et 8) s'étendant radialement depuis le ventilateur ou l'hélice,
**caractérisé en ce que** lesdits canaux d'air (3) sont coplanaires au plan de rotation du ventilateur ou de l'hélice, de manière correspondant au nombre de liquides volatils, et sont placés de telle sorte que le courant d'air s'écoule à travers eux, chaque canal conduisant à un organe (9) d'émission de liquide volatil différent puis à l'atmosphère ; et
(c) des moyens (4) de blocage et de réouverture des canaux, et d'obstruction de l'écoulement d'air à travers eux étant prévus dans chaque canal entre la source et l'organe émetteur de liquide volatil.

2. Appareil selon la revendication 1, dans lequel le nombre de canaux (3 et 8) est compris entre 3 et 8, de préférence entre 4 et 6.

3. Appareil selon la revendication 1, dans lequel l'organe émetteur de liquide (9) comprend une surface d'évaporation, de préférence une mèche s'étendant dans le chemin du courant d'air.

4. Appareil selon la revendication 1, dans lequel les moyens de blocage (4) sont actionnés de manière électromagnétique.

5. Appareil selon la revendication 4, dans lequel les moyens de blocage sont des portes actionnées par des interrupteurs à solénoïde (16).

6. Appareil selon la revendication 1, dans lequel toutes les fonctions de l'appareil peuvent être régulées directement par des moyens de commande sur l'appareil.

7. Appareil selon la revendication 1, dans lequel toutes les fonctions peuvent être régulées à distance, de préférence par transmission radio.

8. Procédé de dissémination dans une atmosphère d'au moins un liquide volatil sélectionné parmi au moins trois liquides prévus dans l'appareil selon les revendications 1 à 7, comprenant le fait de faire passer un courant d'air depuis la source de courant d'air (2) à travers la pluralité de canaux d'air (3 et 8) dont le nombre correspond au nombre de liquides volatils.
